# EUROPEAN PATENT APPLICATION

(11) **EP 2 979 686 A1**
(43) Date of publication of application: **03.02.2016**
(21) Application number: 14772737.4
(22) Date of filing: 26.03.2014
(51) Int. Cl.: A61K 8/81, A45D 29/18, A45D 31/00, A61Q 3/02, A61Q 3/04

(54) **ARTIFICIAL NAIL COMPOSITION, ARTIFICIAL NAIL, METHOD FOR FORMING ARTIFICIAL NAIL, METHOD FOR REMOVING ARTIFICIAL NAIL, AND NAIL ART KIT**

(30) Priority: 29.03.2013 JP 2013073037
(71) Applicant: FUJIFILM Corporation, Tokyo 106-8620 (JP)
(72) Inventor: WATANABE Yukie, Haibara-gun Shizuoka 421-0396 (JP); OOHASHI Hidekazu, Haibara-gun Shizuoka 421-0396 (JP); ABE Junya, Haibara-gun Shizuoka 421-0396 (JP)
(74) Representative: Hoffmann Eitle
(86) International application number: PCT/JP2014/058408
(87) International publication number: WO 2014/157271

(57) **Abstract**

An artificial nail composition comprises (Component A) an ethylenically unsaturated group- and primary amino group- and/or secondary amino group-containing compound, and (Component B) a photopolymerization initiator. Furthermore, Component A is preferably a specific compound represented by Formula (I) and/or a compound represented by Formula (II)

## Description

The present invention relates to an artificial nail composition, an artificial nail, a method for forming an artificial nail, a method for removing an artificial nail, and a nail art kit.

In recent years, nail art, in which a design is applied to fingernails or toenails, has become popular, and various techniques such as decorating a nail (a real nail) with a colored cosmetic composition and attaching an artificial nail made of a synthetic resin (a false nail, a nail tip, etc.) to a real nail have been proposed.

Patent Document 1 discloses a cosmetic makeup composition comprising a film-forming polymer and a colored material in a medium that is acceptable in a cosmetic, the colored material having two substantially flat faces, a thickness in the range of 40 to 200 µm, and a length in the range of 0.1 to 4 mm, and comprising at least one colored polymer film piece that is insoluble in the medium.

Patent Document 2 discloses an artificial nail that is adapted to conform to various finger sizes and shapes, comprising (a) a polymer main body having an upper face and a lower face and having a nail shape and (b) a layer of a deformable material that is attached to at least part of the lower face, the layer being adapted to conform to an upper face of a real nail when attached to the real nail.

Patent Document 3 discloses an artificial nail composition for use in forming an artificial nail by curing with UV irradiation, removal with an acidic solution having a pH of no greater than 3.5 being enabled due to the composition comprising an ionic monomer that is polymerizable by irradiation with UV, in particular a specific acid-responsive monomer (e.g. 2-dimethylaminoethyl methacrylate, 3-dimethylaminopropylacrylamide, etc.).
Patent Document 1 JP-A-2000-256134 (JP-A denotes a Japanese unexamined patent application publication)
Patent Document 2 JP-A-2004-275736
Patent Document 3 JP-A-2009-126833

The present inventors have found that, when a conventional artificial nail composition is stored in an unused state, its stability over time is not sufficient, and as a result of an intensive investigation the present invention has been accomplished.

It is an object of the present invention to provide an artificial nail composition having excellent stability over time and excellent surface gloss, removability, adhesion, and water resistance of an artificial nail obtained thereby, an artificial nail employing the artificial nail composition, a method for forming an artificial nail, a method for removing an artificial nail, and a nail art kit.

The problems of the present invention described above have been solved by the means described in following <1> and <7> to <12>. Preferred embodiments <2> to <6> will also be described below.
<1> An artificial nail composition comprising (Component A) an ethylenically unsaturated group- and primary amino group- and/or secondary amino group-containing compound, and (Component B) a photopolymerization initiator,
<2> the artificial nail composition according to <1>, wherein Component A is a compound represented by Formula (I) and/or a compound represented by Formula (II) 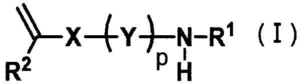 wherein in the Formula R¹ denotes a hydrogen atom, an alkyl group, a cycloalkyl group, or an aryl group, R² denotes a hydrogen atom or a methyl group, p denotes an integer of 0 to 50, X denotes a linking group selected from the group consisting of an alkylene group, -COO-, -CONH-, and -C₆H₄-, Y denotes a divalent linking group, a plurality of Ys may be identical to or different from each other, and Y and R¹ may be bonded to form a ring, 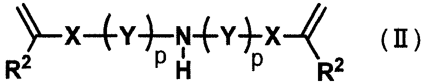 wherein in the Formula R² denotes a hydrogen atom or a methyl group, the two ps independently denote an integer of 0 to 50, X denotes a linking group selected from the group consisting of an alkylene group, -COO-, -CONH-, and -C₆H₄-, Y denotes a divalent linking group, and a plurality of Ys may be identical to or different from each other,
<3> the artificial nail composition according to <1> or <2>, wherein it is a radiation-curable artificial nail composition,
<4> the artificial nail composition according to <2>, wherein in Formula (I) and/or Formula (II) X denotes -COO- or -CONH-, and Y denotes an alkylene group, an alkyleneoxy group, an alkylene amide group, or a group formed by combining same,
<5> the artificial nail composition according to any one of <1> to <4>, wherein Component B is a photopolymerization initiator selected from the group consisting of an acetophenone compound and a phosphine oxide compound,
<6> the artificial nail composition according to any one of <1> to <5>, wherein it further comprises a film-forming agent,
<7> a method for forming an artificial nail, comprising a step of forming a cured film by irradiating the artificial nail composition according to any one of <1> to <6> with actinic radiation,
<8> a method for forming an artificial nail, comprising a step of preparing the artificial nail composition according to any one of <1> to <6> and a step of forming a cured film by irradiating the artificial nail composition with actinic radiation,
<9> an artificial nail formed by curing the artificial nail composition according to any one of <1> to <6> by irradiation with actinic radiation above a human or animal nail or a synthetic support,
<10> a method for removing an artificial nail, comprising a step of removing a cured film of the artificial nail composition according to any one of <1> to <6> by contacting it with an acidic aqueous solution having a pH of no greater than 5.0,
<11> a method for removing an artificial nail, comprising a step of preparing a cured film of the artificial nail composition according to any one of <1> to <6> and a step of removing the cured film by contacting it with an acidic aqueous solution having a pH of no greater than 5.0,
<12> a nail art kit comprising the artificial nail composition according to any one of <1> to <6> and an acidic aqueous solution having a pH of no greater than 5.0.

In accordance with the present invention, there can be provided an artificial nail composition having excellent stability over time and excellent surface gloss, removability, adhesion, and water resistance of an artificial nail obtained thereby, an artificial nail employing the artificial nail composition, a method for forming an artificial nail, a method for removing an artificial nail, and a nail art kit.

The present invention is explained in detail below.

In the present specification, the notation 'xx (lower limit) to yy (upper limit)' means a numerical range that includes xx and yy. The same applies to 'yy (upper limit) to xx (lower limit)'. Furthermore, '(Component A) an ethylenically unsaturated group- and primary amino group- and/or secondary amino group-containing compound', etc. is also called simply 'Component A', etc.

In the present invention, 'mass%' has the same meaning as 'wt%', and 'parts by mass' has the same meaning as 'parts by weight'.

In the present specification, the representation of a group in a compound shown by a formula is as follows. When it is not clearly stated whether the group is substituted or unsubstituted, if the group can have a further substituent it includes not only the unsubstituted group but also a substituted group unless otherwise specified. For example, in the explanation of a formula, if it is stated that 'R denotes an alkyl group or an aryl group', this means that 'R denotes an unsubstituted alkyl group, a substituted alkyl group, an unsubstituted aryl group, or a substituted aryl group'.

Furthermore, in the present specification, (meth)acrylic denotes a concept that includes both acrylic and methacrylic, and the same applies to (meth)acrylate, etc.

In the present invention, a combination of two or more preferred embodiments is a more preferred embodiment.

Moreover, the compound in the present specification includes a low-molecular-weight (molecular weight less than 1,000) molecule and a molecule of an oligomer region (molecular weight at least 1,000 but less than 5,000). Furthermore, the polymer includes a homopolymer and copolymer having a weight-average molecular weight of at least 5,000, and also includes a molecule of an oligomer region (weight-average molecular weight at least 1,000 but less than 5,000).

### (Artificial nail composition)

The artificial nail composition related to the present invention comprises (Component A) an ethylenically unsaturated group- and primary amino group- and/or secondary amino group-containing compound, and (Component B) a photopolymerization initiator.

Component A is preferably a compound represented by Formula (I) and/or a compound represented by Formula (II).

The 'artificial nail composition' referred to here includes both an embodiment in which it is used for the purpose of decorating or protecting a human nail and it is used directly on the human nail as a cosmetic and an embodiment of a false nail in which it is formed into an appropriate nail shape for the application of decoration and it is attached to a human nail, etc.

A subject to which the artificial nail composition of the present invention is applied includes an animal nail as well as a human nail (a real nail, a natural nail) and the composition is also applied to a synthetic resin piece, etc. having any shape for the purpose of decorating and/or protecting a nail.

In addition, the 'human and animal nail and a synthetic resin piece' are also called simply a 'human nail, etc.' or a 'nail'.

The artificial nail in the present invention denotes a layer formed for the purpose of decoration and/or protection above a human or animal nail. The artificial nail includes a resin substrate having any shape for the purpose of decoration and/or protection of a nail.

The explanation below is given mainly in relation to an embodiment (gel nail) in which an artificial nail is formed as a layer above a human nail, etc. by curing the artificial nail composition by irradiation with actinic radiation, but needless to say an 'artificial nail' can be used widely for human nails, etc. including false nails.

As a result of an intensive investigation by the present inventors, it has been found that an artificial nail composition comprising (Component A) an ethylenically unsaturated group- and primary amino group- and/or secondary amino group-containing compound and (Component B) a photopolymerization initiator as essential components has excellent stability over time prior to curing, and an artificial nail obtained by curing this composition has excellent surface gloss, removability, adhesion, and water resistance, and the present invention has thus been accomplished.

Furthermore, an artificial nail formed using the artificial nail composition of the present invention can be removed by a conventional removal method using an organic solvent, but can also be easily removed with an acidic aqueous solution having a pH of no greater than 5.0.

The shape of the artificial nail is not particularly limited, and it may be formed into a desired shape. For example, it may be formed so as to cover a nail surface, may be formed only above part of a nail, or may be formed into a shape that is larger than a nail in order to extend the nail using a nail form, etc.

Furthermore, the thickness of the artificial nail composition of the present invention can be controlled by coating. The thickness is not particularly limited as long as it is in a range that is usually possessed by an artificial nail, but is preferably in the range of 20 to 1,500 µm from the viewpoint of practicality and removability.

The 'artificial nail' in the present invention denotes a cured layer formed above a human nail, etc. for the purpose of decoration or protection by exposing it to actinic radiation, as necessary, after carrying out drying.

The artificial nail composition of the present invention may be used suitably as any of a primer layer, a base layer, a color layer, and/or a top layer of an artificial nail.

Among them, from the viewpoint of removability it is preferable for a layer formed using the artificial nail composition of the present invention to be in contact with a nail.

Furthermore, an upper layer (face on the reverse side to a nail) or a lower layer (face between itself and a nail) of an artificial nail layer formed using the artificial nail composition of the present invention may comprise, separately, a primer layer, a base layer, a color layer, and/or a top layer for the purpose of imparting color, gloss, and adhesion.

The artificial nail composition of the present invention may be suitably used as a radiation-curable artificial nail composition (an 'artificial nail composition for a gel nail').

Furthermore, the artificial nail composition of the present invention has excellent removability even when curing is carried out by exposure to actinic radiation.

A photocurable artificial nail composition is an artificial nail composition that can be cured by actinic radiation. The 'actinic radiation' referred to here is actinic radiation that can give energy that generates an initiating species in the artificial nail composition by irradiation therewith, and includes UV, visible light, etc.

Essential components and optional components used in the artificial nail composition of the present invention are explained below.

The artificial nail composition of the present invention comprises (Component A) an ethylenically unsaturated group- and primary amino group- and/or secondary amino group-containing compound and (Component B) a photopolymerization initiator as essential components and may comprise, in addition thereto, an optional component that is contained in a general artificial nail composition.

Essential components and optional components used in the artificial nail composition of the present invention are explained below.

The artificial nail composition of the present invention comprises (Component A) an ethylenically unsaturated group- and primary amino group-and/or secondary amino group-containing compound and (Component B) a photopolymerization initiator as essential components and may comprise, in addition thereto, an optional component that is contained in a general artificial nail composition. 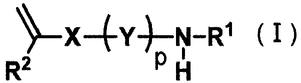 wherein in the Formula R¹ denotes a hydrogen atom, an alkyl group, a cycloalkyl group, or an aryl group, R² denotes a hydrogen atom or a methyl group, p denotes an integer of 0 to 50, X denotes a linking group selected from the group consisting of an alkylene group, -COO-, -CONH-, and -C₆H₄-, Y denotes a divalent linking group, a plurality of Ys may be identical to or different from each other, and Y and R¹ may be bonded to form a ring, 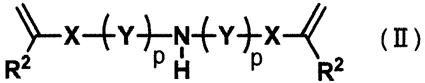 wherein in the Formula R² denotes a hydrogen atom or a methyl group, the two ps independently denote an integer of 0 to 50, X denotes a linking group selected from the group consisting of an alkylene group, -COO-, -CONH-, and -C₆H₄-, Y denotes a divalent linking group, and a plurality of Ys may be identical to or different from each other.

The compound represented by Formula (I) is first explained.

R¹ denotes a hydrogen atom, an alkyl group, a cycloalkyl group or an aryl group. The alkyl group that R¹ can denote is preferably an alkyl group having 1 to 6 carbons, and examples thereof include a methyl group, an ethyl group, an isopropyl group, a *t*-butyl group and an n-hexyl group. The cycloalkyl group that R¹ can denote is preferably a cycloalkyl group having 3 to 6 carbons, and more preferably a cyclopentyl group or a cyclohexyl group.

The aryl group is preferably an aryl group having 6 to 12 carbons, and more preferably a phenyl group.

When Y and R¹ are joined to form ring, the ring is preferably a piperidine ring or a pyrrolidine ring.

R¹ may have a substituent where allowable; the type thereof is not particularly limited, and it is preferably a group that is inactive toward radical polymerization, and more preferably a hydroxy group.

R¹ is preferably a hydrogen atom or an alkyl group having 1 to 6 carbons, more preferably a hydrogen atom or an alkyl group having 1 to 3 carbons, and particularly preferably a hydrogen atom, a methyl group, an ethyl group, or a hydroxyethyl group.

R² denotes a hydrogen atom or a methyl group.

p is a repeating number and represents an integer from 0 to 50, preferably from 0 to 30, more preferably 0 to 20 and yet more preferably 0 to 10.

X denotes a linking group selected from the group consisting of an alkylene group, -COO-, -CONH- and -C₆H₄-. The alkylene group is preferably an alkylene group having 1 to 4 carbons, more preferably a methylene group or an ethylene group and more preferably a methylene group. X is preferably an alkylene group, -COO- or -CONH-, and particularly preferably -CONH-.

Y denotes a divalent linking group, and when p is 2 or more, a plurality of Ys may be identical to or different from each other. Y and R¹ may be bonded to form a ring. Y preferably denotes an alkylene group, an alkyleneoxy group, an alkylene amino group, carbonyl or a group formed by combining same.

The alkylene group is preferably an alkylene group having 1 to 10 carbons, more preferably an ethylene group or an propylene group. The alkyleneoxy group is preferably an ethyleneoxy group. Among them, Y is particularly preferably an ethylene group, a propylene group, an ethyleneoxy group, a propyleneoxy group or a butyleneamide group.

Furthermore, the carbonyl group preferably forms a terminal amide group by bonding with the carbon atom of ethylenically unsaturated group.

Then, the compound represented by Formula (II) is explained.

R², X, Y and p have the same meaning as that of those in Formula (I), and a preferred range is also the same.

Formula (I) is a monofunctional compound that has only one radically polymerizable ethylenically unsaturated group, and Formula (II) is a difunctional compound having two radically polymerizable ethylenically unsaturated groups. In the present invention, it is preferable to use either a compound of Formula (I) or Formula (II) on its own or a monofunctional compound represented by Formula (I) and a difunctional compound represented by Formula (II) in combination. Since when the percentage of the monofunctional compound used is high, the removability of a cured film is excellent, whereas when the percentage of the difunctional compound used is high, the curability is excellent, it is preferable to adjust the curability, adhesion, and removability as appropriate by formulating the two compounds represented by Formula (I) and Formula (II) at an appropriate ratio.

The higher the amine value of a compound represented by Formula (I) and Formula (II), the better the adhesion and removability of a cured film obtained.

Furthermore, the amine value of a nonvolatile component of the artificial nail composition related to the present invention is preferably 0.4 to 8.5 mmol/g, more preferably 1.3 to 8.5 mmol/g, and yet more preferably 1.3 to 6.0 mmol/g. When in this range, removability and water resistance of an artificial nail obtained are superior. The 'nonvolatile component' referred to here means a component other than a volatile solvent (Component E) when it is contained, and corresponds substantially to Components A to D.

As a method for measuring amine value, for example, it may be determined by weighing a sample in a beaker, adding acetic acid thereto, stirring so as to dissolve it, adjusting the measurement temperature to 25°C, and then carrying out titration by means of a titrator using a 0.1 N perchloric acid acetic acid solution as a titration reagent.

The amine value is the amount of perchloric acid consumed during titration and is expressed as the number of moles per g of a sample (nonvolatile component).

The component A of this invention is available as commercial products or is synthesized by a method known to a person skilled in the art. Synthetic example of compound example A-14 is shown later.

Specific examples of component A are shown below, but the present invention is not limited by these examples.

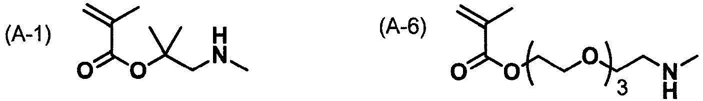

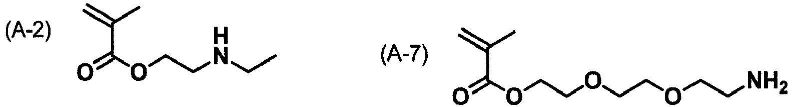

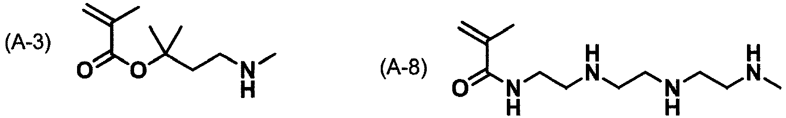

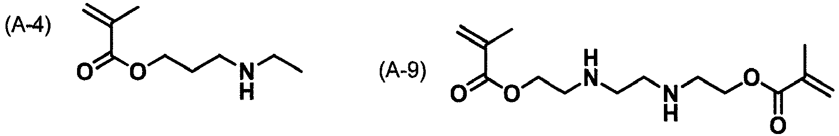

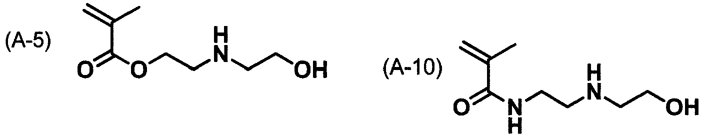

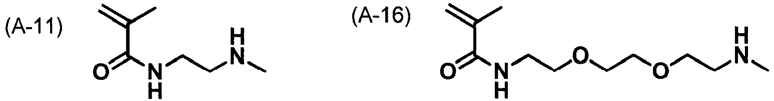

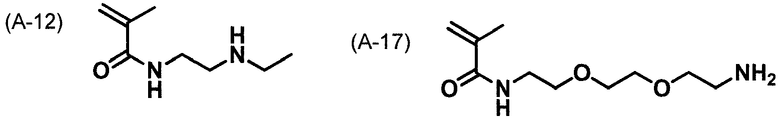

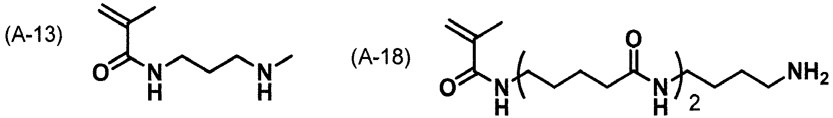

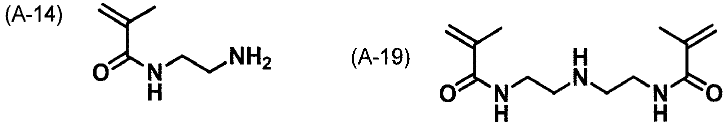

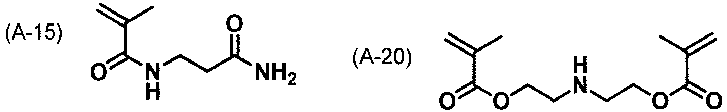

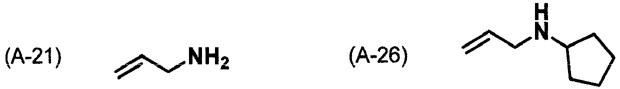

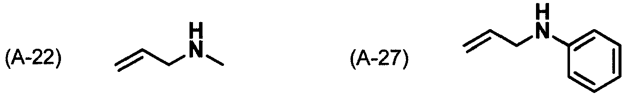

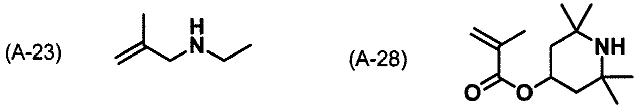

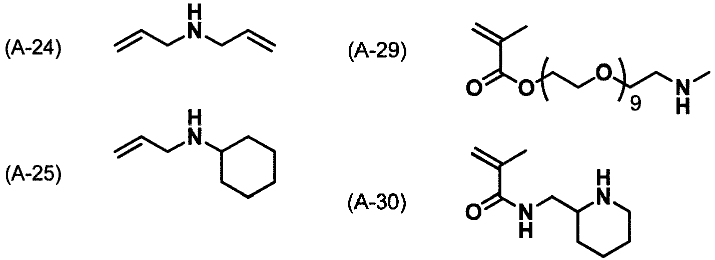

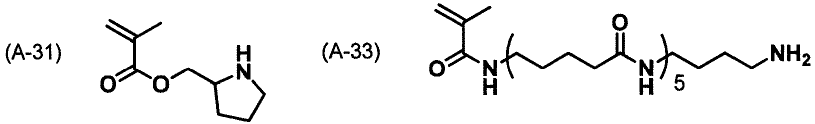

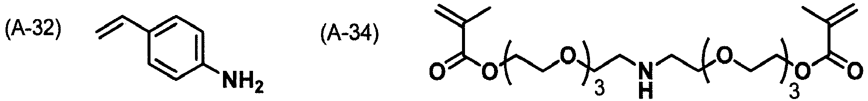

The content of component A in the artificial nail composition of the present invention is preferably 5 to 80 mass % relative to the total mass of non-volatile content of composition, and more preferably 10 to 60 mass %.

### <(Component B) Photopolymerization initiator>

The artificial nail composition of the present invention comprises (Component B) a photopolymerization initiator as an essential component. Due to it comprising Component B, the artificial nail composition of the present invention has photocurablility.

Examples of the photopolymerization initiator include a radical photopolymerization initiator and a cationic photopolymerization initiator, and a radical photopolymerization initiator is preferable.

As the photopolymerization initiator, a known photopolymerization initiator may be used. With regard to the photopolymerization initiator that can be used in the present invention, one type may be used on its own or two or more types may be used in combination. Furthermore, a radical photopolymerization initiator and a cationic photopolymerization initiator may be used in combination.

The photopolymerization initiator that can be used in the present invention is a compound that absorbs actinic radiation and forms a polymerization-initiating species. Examples of actinic energy include γ-rays, β-rays, an electron beam, UV, visible light, and infrared, and UV and/or visible light are preferable.

As the photopolymerization initiator that can be used in the present invention, those below can be cited as preferred examples. But the present invention should not be construed as being limited thereto.

An acetophenone compound (for example, 1-hydroxycyclohexylphenylketone, acetophenone, 2,2-dimetoxy-2-phenylacetophenone, 2,2-dietoxyacetophenone, 2-hydroxy-2-methylpropiophenone, 4'-isopropyl-2-hydroxy-2-metylpropiophenone, 2-metyl-1-[4-(methylthio)phenyl]-2-morphorino-1-propane, 2-benzyl-2-dimethylamino-1-(4-morphorinophenyl)-butane-1-one, benzoin, benzoinmethylether, benzoinethylether or benzoinpropyl ether etc.);
a benzophenone compound (for example, benzophenone, 4.4'-bis(dimethylamino)benzophenone or 3,3-dimethyl-4-metoxy-benzophenone etc.);
a anthraquinone compound (for example, anthraquinone, 2-methylantraquinone, 2-ethylanthraquinone or *tert*-butylanthraquinone etc.);
a thioxanthone compound (for example, 2-chrolothioxantone, diethylthioxanthone, isopropylthioxanthone or diisopropylthioxanthone etc.);
a trihaloalkyl compound (for example, 2,4,6-(trichrolomethyl)triazine, 2,4-trichrolomethyl-6-(4-metoxyphenyl)triazine or tribromomethylphenylsulfone etc.);
a lophin dimer compound (for example, 2-(o-chrolophenyl)-4,5-diphenylimidazoledimer etc.);
an acridine compound (for example, 9-phenylacridine, 1,7-bis(9-acridinyl)heptane, 1,5-bis(9-acridinyl)pentane or 1,3-bis(9-acridinyl)propane etc.);
a phosphineoxide compound (for example, trimethylbenzoyldiphenylphosphineoxide, bis(2,4,6-trimethylbenzoyl)phenylphosphineoxide etc.);
a metallocene compound (for example, biscyclopentadienyl-bis(difluor-pyrryl-phenyl)titanium etc.);
an onium salt (for example, bis(4-*t*-butylphenyl)iodoniumtosylate or triphenylsulfoniumtosylate etc.);
an oxime ester compound (for example, 1-(4-phenylthiophenyl)-1,2-octandion-2-(O-benzoyloxime), 1-[9-ethyl-6-(2-methylbenzoyl)-9H-carbazol-3-il]-ethanone-1-(*O-*acetyloxime) etc.).

Among them, preferred examples of Component B in the present invention include the photopolymerization initiator selected from the group consisting of an acetophenone compound, a phosphineoxide compound, a metallocene compound and a lophin dimer compound, more preferably the photopolymerization initiator selected from the group consisting of an acetophenone compound and a phosphineoxide compound

The content of component B in the artificial nail composition of the present invention is preferably 0.01 to 20 mass % relative to the total mass of non-volatile content of artificial nail composition, more preferably 0.1 to 15 mass % and yet more preferably 0.5 to 12 mass %. When in this range, the curability of artificial nail composition is better, and adhesion, gloss and water resistance of an artificial nail that is obtained are excellent.

The artificial nail composition of the present invention preferably comprises (Component C) a film-forming agent as an optional component.

### <(Component C) Film-forming agent>

The artificial nail composition of the present invention preferably comprises a film-forming agent.
The film-forming agent means the material which forms a coating having singly self-supporting properties when formed above a substrate, and the film-forming agent is preferably a film-forming polymer having a weight-average molecular weight of at least 5,000. The film-forming agent may have an ethylenically double bond in a side chain.

The kind of a film-forming polymer is not particularly limited, but any known polymer such as an acrylic polymer, a urethane polymer, a cellulosic polymer, an ester polymer, an amide polymer, a vinyl polymer, an ether polymer, a styrenic polymer, a carbonate polymer, a urea polymer and an ethylene polymer may be used.

Among them, from the viewpoint of film-formablity and ease of handling (viscosity) aspects, an ether polymer, an ethylene polymer (specifically, polyethyleneimine polymer), a urethane polymer and acrylic polymer are preferable, a urethane polymer and acrylic polymer are more preferable, and acrylic polymer is yet more preferable.

With regard to the acrylic polymer, any polymer may be used suitably as long as it is a polymer obtained by polymerization of a known acrylic acid derivative (e.g. an acrylic acid ester such as methyl acrylate or ethyl acrylate, an acrylic acid amide such as acrylamide or acryloylmorpholine) or a methacrylic acid derivative (e.g. a methacrylic acid ester such as methyl methacrylate or ethyl methacrylate, a methacrylic acid amide such as methacrylamide or methacrylic acid isopropylamide). In the present invention, an acrylic polymer containing an addition-polymerizable curable group (e.g. a (meth)acrylic group, etc.) in a side chain is particularly preferably used. However, it should not be construed as being limited to these examples.

With regard to the urethane-based polymer, any polymer may be used suitably as long as it is a polyurethane formed from a known polyisocyanate compound (e.g. tolylene diisocyanate, isophorone diisocyanate, etc.) and a known polyol compound (e.g. an alkylenediol such as butanediol or hexanediol, an arylenediol such as p-hydroxystyrene, a polyether diol such as polyethylene glycol or polypropylene glycol, a polyester diol such as polyethylene glycol terephthalate, a polycarbonate diol such as polyethylene glycol carbonate, etc.). However, it should not be construed as being limited to these examples.

With regard to the the cellulosic polymer, any cellulosic polymer such as carboxymethylcellulose, nitrocellulose, triacetylcellulose etc. may be used suitably. However, it should not be construed as being limited to these examples.

With regard to the ester-based polymer, any polyester may be used suitably as long as it is a polyester formed from a known polycarboxylic acid compound (e.g. succinic acid, adipic acid, phthalic acid, etc.) and a known polyol compound. Furthermore, a polyester formed from a hydroxycarboxylic acid compound such as polylactic acid may also be suitably used. However, it should not be construed as being limited to these examples.

With regard to the amide-based polymer, any polyamide may be used suitably as long as it is a polyamide formed from a known polycarboxylic acid compound and a known polyamine compound (e.g. ethylenediamine, phenylenediamine, etc.). Furthermore, a polyamino acid, which is a protein formed from amino acids, may also be used suitably. However, it should not be construed as being limited to these examples.

With regard to the vinyl-based polymer, any vinyl-based polymer may be used suitably as long as it is a vinyl-based polymer obtained by polymerization of a known vinyl compound (e.g. vinyl acetic acid, vinyl chloride, butadiene, etc.). However, it should not be construed as being limited to these examples.

With regard to the ether-based polymer, any polyether may be used suitably as long as it is a polyether (e.g. polyethylene glycol, polypropylene glycol, etc.) formed from a known polyol compound. However, it should not be construed as being limited to these examples.

With regard to the styrene-based polymer, any polystyrene may be used suitably as long as it is a polystyrene formed from a known styrene compound (e.g. styrene, 4-carboxystyrene, 4-acetoxystyrene, etc.). However, it should not be construed as being limited to these examples.

With regard to the carbonate-based polymer, any polycarbonate may be used suitably as long as it is a polycarbonate formed from a known carbonic acid derivative (e.g. phosgene, dimethyl carbonate, dimethyl carbonate, etc.) and a known polyol compound. However, it should not be construed as being limited to these examples.

With regard to the urea-based polymer, any polyurea may be used suitably as long as it is a polyurea formed from a known polyisocyanate compound and a known polyamine compound. However, it should not be construed as being limited to these examples.

The content of Component C in the artificial nail composition of the present invention is preferably 0 to 80 mass % relative to the total mass of non-volatile content of artificial nail composition and more preferably 20 to 60 mass %.

The artificial nail composition of the present invention may comprise as an optional component (Component D) a polymerizable compound containing no amino group.

### <(Component D) Polymerizable compound containing no amino group>

The artificial nail composition of the present invention may comprise, in addition to Component A, a polymerizable compound containing no amino group as Component D, and it is preferably a radically polymerizable ethylenic compound. Component D is an addition-polymerizable compound that does not contain any of a primary amino group, a secondary amino group, or a tertiary amino group, and due to it comprising Component D the artificial nail composition of the present invention may be suitably used as a photocurable artificial nail composition.

Furthermore, when Component D is added to the artificial nail composition of the present invention, the total amount thereof added is preferably smaller than the amount of Component A added and is preferably 0 to 50 mass% of the total nonvolatile components of the artificial nail composition. By adding at least one type of Component D to Component A, the artificial nail composition of the present invention may be used more suitably as a photocurable artificial nail composition.

Examples of the radically polymerizable ethylenic compound include unsaturated carboxylic acids such as acrylic acid, methacrylic acid, itaconic acid, crotonic acid, isocrotonic acid, and maleic acid, and salts thereof, an ethylenically unsaturated group-containing anhydride, acrylonitrile, styrene and, furthermore, radically polymerizable compounds like oligomers such as various types of unsaturated polyesters, unsaturated polyethers, unsaturated polyamides, and unsaturated urethane.

Examples of other polymerizable compound include an acrylic derivatives such as 2-ethylhexyl(meth)acrylate, 2-hydroxyethyl(meth)acrylate, butoxyethyl(meth)acrylate, carbitol (meth)acrylate, cyclohexyl (meth)acrylate, benzyl (meth)acrylate, methyl (meth)acrylate, ethyl (meth)acrylate, n-propyl (meth) acrylate, isopropyl (meth)acrylate, n-butyl (meth)acrylate, allyl (meth)acrylate, glycidyl (meth)acrylate, N-methylol (meth)acrylamide, diacetone (meth)acrylamide, and epoxy (meth)acrylate, allyl compound derivatives such as allyl glycidyl ether, diallyl phthalate, triallyl trimellitate, etc., and more specifically, commercially available or industrially known radically polymerizable or crosslinking monomers and oligomers, such as those described in 'Kakyozai Handobukku' (Crosslinking Agent Handbook), Ed. S. Yamashita (Taiseisha, 1981); 'UV/EB Koka Handobukku (Genryo)' (UV/EB Curing Handbook (Starting Materials)) Ed. K. Kato (Kobunshi Kankoukai, 1985); 'UV/EB Koka Gijutsu no Oyo to Shijyo' (Application and Market of UV/EB Curing Technology), p. 79, Ed. RadTech (CMC, 1989); and E. Takiyama 'Poriesuteru Jushi Handobukku' (Polyester Resin Handbook), (The Nikkan Kogyo Shimbun Ltd., 1988) may be used.

From the viewpoint of removability, adhesion and gloss of an artificial nail that is obtained, preferred examples of Component D include an ethylenically unsaturated compound having hydroxy group or an ethylenically unsaturated compound having (poly)alkyleneoxy group, more preferably a (meth)acrylate compounds having hydroxy group or a (meth)acrylate compounds having (poly)alkyleneoxy group, yet more preferably a (meth)acrylate compounds having hydroxy group and especially preferably a monofunctional (meth)acrylate compounds having hydroxy group.

Preferred examples of a (meth)acrylate compounds having hydroxy group include hydroxyalkyl(meth)acrylate such as 2-hydoxyethyl(meth)acrylate, 2-hydroxypropyl(meth)acrylate, 3-hydroxypropyl(meth)acrylate, 2,3-dihydroxypropyl(meth)acrylate and 4-hydroxybutyl(meth)acrylate, polyethyleneglycolmono(meth)acrylate, polypropyleneglycolmono(meth)acrylate, poly(ethyleneglycol/propyleneglycol)-mono(meth)acrylate, polyethyleneglycol/polypropyleneglycol-mono(meth)acrylate, poly(ethyleneglycol/tetramethyleneglycol)-mono(meth)acrylate, poly(propyleneglycol/tetramethyleneglycol)-mono(meth)acrylate and polypropyleneglycol/polybuthyleneglycol-mono(meth)acrylate.

Among them, preferred examples of Component D include a hydroxyalkyl(meth)acrylate compound, more preferably 2-hydroxyethyl(meth)acrylate, 2-hydroxypropyl(meth)acrylate or 3-hydroxypropyl(meth) acrylate and yet more preferably 2-hydroxyethyl(meth)acrylate.

The artificial nail composition of the present invention may comprise as an optional component (Component E) a solvent.

### <(Component E) Solvent>

The artificial nail composition of the present invention may comprise (Component E) a solvent from the viewpoint of coating properties.

Any solvent may be used suitably as long as it is a known organic solvent for Component C. Examples include an alcohol-based solvent such as ethanol, isopropanol, ethylene glycol monomethyl ether, ethylene glycol monobutyl ether, propylene glycol monomethyl ether, diethylene glycol monoethyl ether, or dipropylene glycol monomethyl ether and solvents based on acetates thereof, an ester-based solvent such as ethyl acetate or butyl acetate, a ketone-based solvent such as acetone, methyl ethyl ketone, methyl isobutyl ketone, or cyclohexanone, and an ether-based solvent such as tetrahydrofuran or methyl *t*-butyl ether. However, it should not be construed as being limited to these examples.

From the viewpoint of drying rate, the boiling point of the solvent is preferably 30°C to 130°C, more preferably 35°C to 100°C, yet more preferably 40°C to 90°C and particularly preferably 50°C to 85°C. When in this range, the drying property and coating property are excellent.

With regard to Component E, one type thereof may be used on its own or two or more types thereof may be used in combination, but a completely curable artificial nail composition that contains substantially no Component E or water is preferable.

When Component E is used, it is preferable for it to comprise an alcohol-based solvent and/or a ketone-based solvent, and it may comprise an alcohol-based solvent and a ketone-based solvent.

When Component E is used in the artificial nail composition of the present invention, the content thereof is not particularly limited but is preferably 0 to 30 mass% relative to the total mass of non-volatile components in the artificial nail composition, more preferably 0 to 20 mass%, and particularly preferably 0 to 10 mass%. When in this range, the coating properties are superior, and an artificial nail obtained has superior removability and adhesion.

Furthermore, the artificial nail composition of the present invention is preferably a completely curable composition that does not contain an organic solvent or water. In this case, Component A and Component D, etc., which are low-molecular-weight molecules, also function as solvents for (Component C) a film-forming agent.

### <Other components>

The artificial nail composition of the present invention may optionally comprise, as an optional component, other than Component A to Component E, an additive component that can usually be added to an artificial nail composition within a range that does not impair the effects of the present invention. Examples of such an additive component include a tertiary amino group-containing polymerizable compound, an antifoaming agent, a buffer agent, a chelating agent, a dispersant, a dye, a filler, a pigment, a preservative, a resin powder (e.g. poly(meth)acrylic acid, etc.), an inorganic powder (e.g. silica gel, etc.), a metal foil, and a wetting agent. However, it should not be construed as being limited to these examples. Furthermore, it preferably does not comprise a tertiary amino group-containing polymerizable compound from the viewpoint of adhesion/water resistance of the artificial nail composition.

These optional components may be added to the artificial nail composition in advance, but a necessary amount of a necessary component may be added to the artificial nail composition each time when carrying out a cosmetic procedure.

Furthermore, the amount of each component in the artificial nail composition of the present invention is not particularly limited, but it is preferable that, as a mass ratio of nonvolatile components in the artificial nail composition, Component A/Component B/Component C/Component D = 50 to 80/0.01 to 20/0 to 80/0 to 50. Here, the total of Components A to D is defined as being 100 parts by mass. When in this range, the coating properties are superior, and the removability and gloss of an artificial nail obtained are superior.

With regard to the artificial nail composition of the present invention, it is preferable for a cured film formed to have an appropriate hardness and an appropriate flexibility.

### (Artificial nail)

The artificial nail of the present invention is an artificial nail that has a layer formed by curing the artificial nail composition of the present invention with actinic radiation, and may be an artificial nail cured by exposure after removing solvent from the artificial nail composition of the present invention by drying prior to exposure of the composition to actinic radiation.

The artificial nail of the present invention is suitable as a gel nail.

With regard to the artificial nail of the present invention, at least part thereof may be formed with the artificial nail composition of the present invention and it may have another layer or structure, or the entirety of the artificial nail may be formed with the artificial nail composition of the present invention.

A layer formed with the artificial nail composition of the present invention may be used suitably for any of a primer layer, a base layer, a color layer, and/or a top layer in the artificial nail of the present invention.

Furthermore, the artificial nail of the present invention may have only one layer of the layer formed with the artificial nail composition of the present invention, or it may have two or more layers thereof.

Among them, from the viewpoint of removability it is preferable for at least one layer formed with the artificial nail composition of the present invention to be in direct contact with a human nail, etc.

Moreover, the thickness of the layer formed with the artificial nail composition of the present invention in the artificial nail of the present invention is not particularly limited but is preferably 10 to 2,000 µm, more preferably 20 to 1,500 µm, and yet more preferably 20 to 1,000 µm.

### (Method for forming artificial nail)

The method for forming an artificial nail of the present invention is not particularly limited as long as it is a method for forming an artificial nail using the artificial nail composition of the present invention, and is preferably a method comprising a step of forming a coated film by coating a human nail, etc. with the artificial nail composition of the present invention, and a step of forming a cured film by irradiating the coated film with actinic radiation.

When forming an artificial nail with the artificial nail composition of the present invention, a method in which, after removing as necessary solvent by drying, the composition is cured by exposing to actinic radiation is suitable.

Furthermore, the artificial nail composition of the present invention preferably comprises, in addition to an ethylenically unsaturated group- and primary amino group- and/or secondary amino group-containing compound (Component A) and a photopolymerization initiator (Component B), at least one type of film-forming agent (Component C). In this case, after preparing the artificial nail composition using a solvent (Component E), it is preferable to remove by distillation Component E from the composition obtained before it is used.

Moreover, the thickness of the layer formed with the artificial nail composition of the present invention in the method for forming an artificial nail of the present invention is not particularly limited but is preferably 10 to 2,000 µm, more preferably 20 to 1,500 µm, and yet more preferably 20 to 1,000 µm.

### <Coating step>

The method for forming an artificial nail of the present invention preferably comprises a step (coating step) of forming a coated film by coating a human nail, etc. with the artificial nail composition of the present invention.

In addition, a nail tip, etc. is not particularly limited for use as long as it is a substrate used in an artificial nail, and coating may be carried out on for example a synthetic resin substrate. In this case, after a coating step and an exposure step, it may be processed into a nail tip shape.

A coating method is not particularly limited; it may be carried out by a known method, and a method in which coating is carried out using a flat brush, a round brush, etc. makes a fine design possible and can be cited as a preferred example. Furthermore, spray coating or inkjet coating may be carried out.

The thickness of a cured film is not particularly limited and the coating thickness may be adjusted as appropriate while taking into consideration a desired thickness for an artificial nail obtained.

### <Exposure step>

The method for forming an artificial nail of the present invention preferably comprises a step (exposure step) of carrying out curing by exposure after the coated film is dried as necessary.

### <Drying step>

When the artificial nail composition of the present invention comprises (Component E) a solvent, it is preferable to first remove the solvent from the composition by drying.

A drying method is not particularly limited, and a known method may be employed.

Specific preferred examples include a method in which drying is carried out by allowing it to stand at room temperature (e.g. 10°C to 30°C), a method in which drying is carried out by allowing it to stand under a flow of gas, a method in which drying is carried out by heating, and a method in which the above methods are combined. A heating method is not particularly limited, and examples include a method in which it is allowed to stand under an atmosphere with a temperature that is higher than room temperature, a method in which it is allowed to stand under a flow of heated gas, and a method in which heating is carried out by means such as a heater or an infrared lamp (an IR lamp).

The drying time is not particularly limited and may be adjusted as appropriate according to the formulation of the artificial nail composition, the drying method, or the coating thickness, but from the viewpoint of simplicity and surface gloss or smoothness being imparted by self-leveling, it is preferably 10 seconds to 20 minutes, more preferably 15 seconds to 10 minutes, yet more preferably 30 seconds to 5 minutes, and particularly preferably 30 seconds to 2 minutes.

### <Exposure means, etc.>

When an artificial nail is formed by irradiation with actinic radiation, the artificial nail composition of the present invention preferably comprises, in addition to (Component A) an ethylenically unsaturated group- and primary amino group-and/or secondary amino group-containing compound and (Component B) a photopolymerization initiator, (Component D) a polymerizable compound containing no amino group.

Examples of light used for exposure include UV and visible light.

The exposure means is not particularly limited; known exposure means may be used, and examples include a UV lamp such as a mercury lamp or a metal halide lamp, a light-emitting diode (LED), and a laser diode (LD).

The exposure time is not particularly limited and is preferably 2 seconds to 15 minutes, more preferably 5 seconds to 10 minutes, and yet more preferably 5 seconds to 5 minutes. Furthermore, exposure may be carried out intermittently, continuously, or pulse-wise, and any method may be used for exposure.

The method for forming an artificial nail of the present invention may comprise a step of washing or wiping the surface of an obtained artificial nail after the drying or exposure step from the viewpoint of gloss or appearance of the obtained artificial nail and removal of uncured component when exposure has been carried out.

Examples of a washing or wiping method include a method in which wiping is carried out using a wiping substrate such as a wiping sheet or a sponge wipe that are made to contain a solvent such as ethanol, and a method in which washing is carried out with water or a solvent such as ethanol.

Furthermore, a solvent used for washing or wiping is preferably a solvent that does not dissolve an obtained artificial nail.

Moreover, the method for forming an artificial nail of the present invention preferably comprises a step of roughening the surface of a human nail, etc. prior to the coating step. With this embodiment, adhesion and durability of an artificial nail (gel nail) obtained by exposure are excellent.

A method for roughening a nail surface is not particularly limited, and it can be carried out by a known method. Preferred examples include a method in which roughening is carried out using a nail sander such as a file.

Furthermore, the method for forming an artificial nail of the present invention may comprise another known step.

### (Method for removing artificial nail)

The method for removing an artificial nail of the present invention comprises a step of carrying out removal by contacting a cured film of an artificial nail composition related to the present invention with an acidic aqueous solution having a pH of no greater than 5.0.

The artificial nail composition of the present invention can be removed by an organic solvent such as acetone, which is a conventional removal liquid, but it can be removed more safely without imposing a burden on the skin and nail by means of an acidic aqueous solution having a pH of no greater than 5.0. A removal liquid suitably used in the present invention preferably comprises an organic/inorganic acid component for making it acidic, water, an optional stabilizer, and an optional surfactant. An embodiment of a preferred removal liquid is explained below.

### <Organic/inorganic acid component>

As the removal liquid, a known inorganic acid (phosphoric acid, hydrochloric acid, etc.) or an organic acid may be used in order to make the aqueous solution acidic. As an acid component used in the removal liquid, an organic acid that is usually contained in food or a food additive is preferably used. Preferred examples of the organic acid include citric acid, gluconic acid, lactic acid, malic acid, an amino acid (aspartic acid, glutamic acid, etc.), and carbonic acid.

### <Water and pH>

The water used in the removal liquid is not particularly limited, and tap water, distilled water, ion-exchanged water, etc. may be used.

The content of the water in the removal liquid is not particularly limited and is preferably 50 to 99.5 mass% relative to the total mass of the removal liquid, more preferably 70 to 99 mass%, and yet more preferably 90 to 99 mass%.

The pH of the removal liquid is, from the viewpoint of the ability to ionize a primary or secondary amino group-containing polymer contained in the artificial nail of the present invention, that is, from the viewpoint of removability, preferably no greater than 5.0, more preferably no greater than 4.0, and yet more preferably no greater than 3.0. Furthermore, the pH of the removal liquid is preferably at least 1.0, and more preferably at least 2.0 from the viewpoint of safety toward the skin and nail.

Furthermore, it is preferable to add an acid salt so as to make a buffer in order to prevent the pH from varying over time or varying due to an external factor such as dilution/concentration, etc. of the liquid. In order to make a buffer, a method in which a salt of the above acid, preferably the sodium salt (sodium citrate, sodium gluconate, sodium carbonate, disodium phosphate) is made to be present at the same time as the acid can be cited, but this should not be construed as being particularly limiting. When the pH conditions are satisfied, a commercially available synthetic lemon water may also be used.

### <Stabilizer>

The removal liquid used in the present invention may optionally contain a stabilizer. Addition of a stabilizer can suppress coloration of the liquid over time, the occurrence of corrosion and mold, etc. The stabilizer is not particularly limited, and a known industrially used preservative, food preservative, pharmaceutical preservative, antioxidant/photostabilizer, etc. may be used.

Among them, as the stabilizer, a parahydroxybenzoic acid ester compound is preferable.

The amount of stabilizer added is not particularly limited and is preferably 0.001 to 10 mass% relative to the total mass of the removal liquid, more preferably 0.005 to 5 mass%, and yet more preferably 0.01 to 2 mass%.

From the viewpoint of increasing the removal speed, the removal liquid is preferably heated before use to a degree that does not impair the safety. The heating is preferably to 30°C to 45°C, more preferably to 35°C to 45°C, and particularly preferably to 40°C to 45°C. Furthermore, when an artificial nail is immersed in a removal liquid, the liquid in which it is immersed may be stirred.

The artificial nail of the present invention may be removed easily by contacting it with an acidic aqueous solution having a pH of no greater than 5.0 in place of a conventionally used organic solvent such as acetone.

A contacting method is not particularly limited; an artificial nail may be directly immersed in a removal liquid, an artificial nail may be wrapped with cotton, etc. that is wetted with a removal liquid, or spraying may be carried out using an atomizer or a shower. However, it should not be construed as being limited to these examples.

Furthermore, the method of removing an artificial nail of the present invention preferably comprises a step of damaging the artificial nail surface and/or the extremity so that part of a layer formed with the artificial nail composition of the present invention is exposed on the surface.

A damaging method is not particularly limited, but a nail sander such as a file may be suitably used.

Specific preferred examples of the removal method are shown below. That is, after the artificial nail surface and/or the extremity is optionally and roughly damaged by rubbing with a nail sander to a degree such that part of a layer formed with the artificial nail composition of the present invention is exposed on the surface layer, then immersed in an acidic aqueous solution having a pH of no greater than 5.0, and allowed to stand for about 1 to 5 minutes to thus change the solubility of the artificial nail, the artificial nail is wiped with a cloth, an unwoven cloth, etc., or peeled off with a pushing force using a tool with a spatula shape or a stick shape, thus enabling it to be peeled off very easily and safely without imposing a burden on the finger tip or nail.

In addition, wiping and peeling with a pushing force can be carried out during immersion, and removal can be carried out more rapidly. Furthermore, wiping/peeling may be promoted by applying ultrasonic waves, vibration, etc.

Moreover, the method for removing an artificial nail of the present invention may comprise another known step.

### (Nail art kit)

The nail art kit of the present invention comprises the artificial nail composition of the present invention and a removal liquid, the removal liquid being preferably an acidic aqueous solution having a pH of no greater than 5.0.

Preferred embodiments of the artificial nail composition of the present invention and the removal liquid in the nail art kit of the present invention are the same as those described above.

Furthermore, the nail art kit of the present invention may comprise an optional product in addition to the artificial nail composition and the removal liquid.

Examples include, but are not limited to, an artificial nail composition for coloring, the top, etc. other than the artificial nail composition of the present invention, a nail sander such as a file, a brush or a fine brush such as a flat fine brush for coating the artificial nail composition, exposure equipment such as a UV light, a liquid for wiping or washing, a wipe for wiping, a nail brush, a dust brush, a nail form used for extending a nail, a decorative stone made of an acrylic, glass, metal, or natural stone, a nail sticker, a decorative powder such as glitter or a hologram, a cutter, a spatula, a stick, and a spacer for keeping fingers spaced in order to prevent nails from contacting each other.

Keratin, which is the main component of nail, is a chain-form macromolecular protein in which amino acids such as cysteine, serine, and glutamic acid are bonded via peptide bonds. Since a large amount of sulfur-containing amino acid such as cysteine is contained, polypeptide chains are bonded to each other via strong cystine bonds (disulfide bonds) to form cystine, which is a dimer of cysteine.

It is surmised that adhesion to a nail and adhesion to an upper layer that is optionally formed are exhibited by formation of crosslinking between the nail surface and the artificial nail composition due to hydrogen bonding. This effect is strongly exhibited due to there being a primary or secondary amino group rather than a tertiary amino group.

Furthermore, it is surmised that degradation over time of adhesion/water resistance of the artificial nail composition itself when a tertiary amino group is used is due to conversion of an amino group into an *N*-oxide. When converted into an *N*-oxide, an unpaired electron of an amino group is lost to thus cause degradation of adhesion and an increase in water solubility, thus degrading water resistance as well. This effect is enhanced particularly when an amino acid that is added is a low-molecular-weight molecule. In the present invention, due to the use of a compound having a primary and/or secondary amine, which is more difficult to convert into an *N*-oxide, these effects are greatly suppressed, and stabilization of performance over time, which could not be solved, has been achieved.

The present invention is more specifically explained by way of Examples below, but the present invention should not be construed as being limited by the modes of these Example. Unless otherwise specified, 'parts' and '%' are on a mass basis. A-1 to A-34 described in the Examples are the same compounds as A-1 to A-34 described above.

With regard to the compound examples, commercially available products (A-21 to 27 and A-32) may be used or they can be easily synthesized from commercially available starting materials.

Compound example A-1 may be synthesized by reacting an amino alcohol with methacrylic acid anhydride to thus effect methacryloylation.

Synthesis of A-14 is illustrated below.

### (Synthesis of A-14)

A three-necked flask was charged with water (63 g), ethylenediamine (8 g), and MeOH (60 g) and cooled so that the internal temperature was below 5°C while stirring. Benzoic acid (32 g) was added thereto, stirring was carried out, and methacrylic acid anhydride (33 g) was added dropwise over 3 hours while maintaining the internal temperature at no greater than 5°C. Subsequently, a reaction was carried out at a reaction temperature of 5°C for 3 hours. 85% phosphoric acid (7.5 g) was added, stirring was carried out, and after confirming that a pH of about 3.3 had been attained, extraction was carried out by adding ethyl acetate (296.5 g) and pure water (91.5 g). The lower layer was collected, washed with ethyl acetate (288.5 g) and separated. The lower layer was collected again, washed with added ethyl acetate (191.5 g), separated, and the lower layer was collected, washed with added n-hexane (164 g), and separated. A liquid taken out from the lower layer was neutralized with triethylamine, and extraction was carried out by adding diethyl ether (300 g). An organic layer obtained by separation was subjected to concentration under vacuum using a rotary evaporator, thus giving A-14 in a yield of 40%.

### <Preparation of artificial nail composition>

### (Examples 1 to 22 and Comparative Examples 1 to 3)

The various components shown in Table 1 below were uniformly mixed, thus giving artificial nail compositions of Examples 1 to 22 and Comparative Examples 1 to 3.

Photopolymerization initiators I-1 to I-6 and ethylenically unsaturated compounds M-1 to M-5 used for preparation of the artificial nail compositions were as follows.

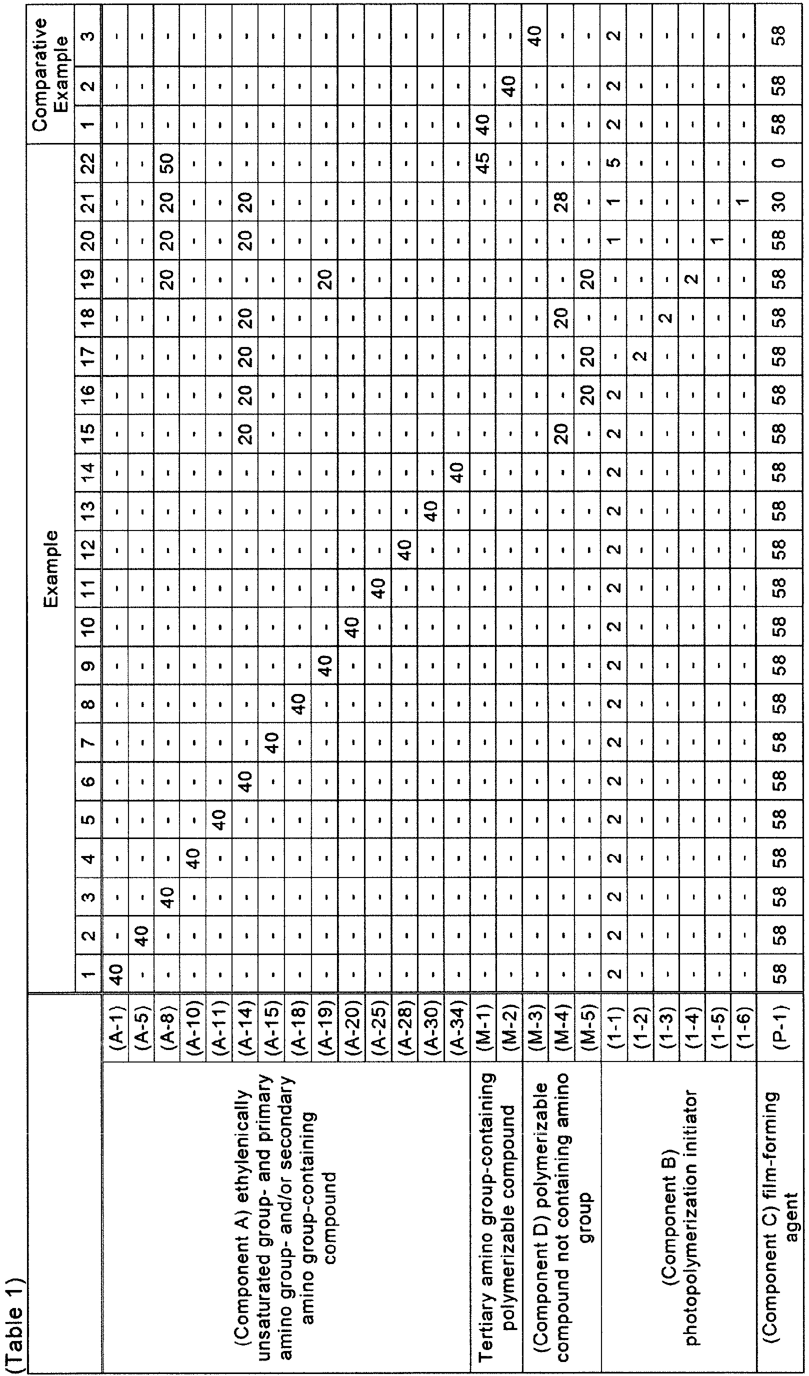

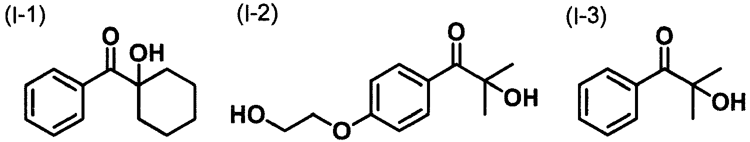

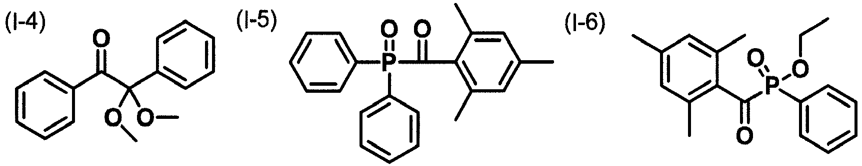

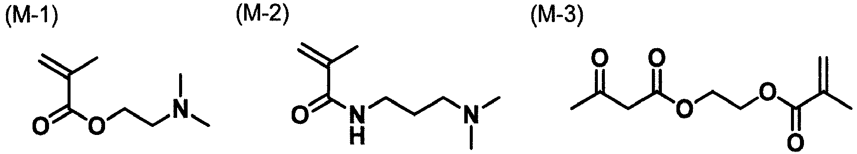

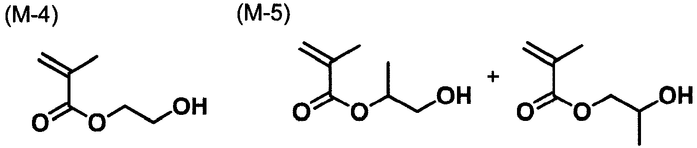

Furthermore, P-1 is urethane (meth)acrylate KUA-PEA2I manufactured by KSM Co., Ltd. KUA-PEA2I is a difunctional acrylate produced from polyether polyol and IPDI.

In addition, I-1 is IRGACURE 184 manufactured by BASF Japan, I-5 is diphenyl(2,4,6-trimethylbenzoyl)phosphine oxide (Wako Pure Chemical Industries, Ltd.), and I-2 to I-4 and I-6 are commercial products.

M-1 to M-5 are reagents manufactured by Tokyo Chemical Industry Co., Ltd.

### <Preparation of removal liquid>

The various components shown in Table 2 below were uniformly mixed, thus giving removal liquids E-1 to E-3.

**(Table 2)**

| Removal liquid | | E-1 | E-2 | E-3 |
|---|---|---|---|---|
| Organic /- inorganic acid component | Citric acid | 2.7 | 2.0 | 1.2 |
| | Trisodium citrate dihydrate | 0.3 | 1.3 | 2.5 |
| | Tap water | 297 | 296.7 | 296.3 |
| Liquid pH | | 2.6 | 3.5 | 4.7 |

Details of the reagents used in the removal liquids are as follows.
Citric acid (Tokyo Chemical Industry Co., Ltd.)
Trisodium citrate dihydrate (Wako Pure Chemical Industries, Ltd.)

### <Formation of artificial nail>

A shaped plastic substrate on a nail was coated with a fixed amount of artificial nail composition obtained using a flat brush and irradiated using a UV lamp (36 W) for 2 minutes. Subsequently, the surface was washed with ethanol, and when the artificial nail thus formed was then visually examined, it had completely solidified. The film thickness at this time was measured and was found to be about 100 µm (±10 µm).

### <Evaluation of artificial nail>

### <Gloss>

Gloss of an artificial nail formed was visually evaluated using the criteria below under the light of a fluorescent lamp.

### (Evaluation criteria)

A: the fluorescent lamp was seen on the nail surface, and the image could be clearly recognized.

B: the fluorescent lamp was seen on the nail surface, and the image could be fairly clearly recognized.

C: the fluorescent lamp was seen on the nail surface, but the image could not be clearly recognized.

### <Removability>

The artificial nail obtained was immersed in a removal liquid at 40°C for 15 seconds and wiped 20 times using kitchen paper soaked with the removal liquid. This immersion-wiping operation was repeated 10 times, and following this the film thickness (µm) remaining of the remaining artificial nail was evaluated.

### <Adhesion>

The uppermost layer of the artificial nail obtained was scraped using an HB pencil with a fixed load, and the presence or absence of the occurrence of peel-off and the presence or absence of scratch marks were evaluated using the evaluation criteria below.
A: neither scratching nor peel-off occurred.
B: peel-off did not occur, but some scratching occurred.
C: peel-off did not occur, but scratching occurred.
D: peel-off occurred.

### <Water resistance>

An artificial nail obtained was placed in tap water at 45°C (pH 6.4), and stirring was carried out at 200 rpm for 5 hours. The artificial nail was taken out after stirring, the surface state was visually examined, and evaluation was carried out using the evaluation criteria below.
A: neither scratching nor peel-off occurred.
B: peel-off did not occur, but some scratching occurred.
C: peel-off did not occur, but scratching occurred.
D: peel-off occurred.

### <Evaluation when aged artificial nail composition was used>

The artificial nail composition was charged into a brown bottle and then stored in a temperature-controlled room at 40°C for 5 days. After storage, an artificial nail was formed by the same method as that of the evaluation method, and tests for <removability>, <adhesion>, <water resistance>, and <gloss> were carried out.

The results are summarized in Table 3.

**(table 3)**

| | Use artificial nail composition immediately after preparation | | | | | | Use aged artificial nail composition | | | | | |
|---|---|---|---|---|---|---|---|---|---|---|---|---|
| | Gloss | Removability | | | Adhesion | Water resistance | Gloss | Removability | | | Adhesion | Water resistance |
| | | Removal liquid E-1 | Removal liquid E-2 | Removal liquid E-3 | | | | Removal liquid E-1 | Removal liquid E-2 | Removal liquid E-3 | | |
| Example 1 | A | 1 | 2 | 5 | A | A | A | 1 | 3 | 5 | A | A |
| Example 2 | A | 0 | 1 | 3 | A | B | A | 3 | 6 | 7 | A | B |
| Example 3 | A | 0 | 0 | 1 | A | A | A | 0 | 1 | 1 | B | A |
| Example 4 | A | 1 | 1 | 3 | A | A | A | 2 | 3 | 4 | B | A |
| Example 5 | A | 2 | 2 | 4 | A | A | A | 1 | 3 | 5 | A | A |
| Example 6 | A | 0 | 0 | 0 | A | B | A | 0 | 1 | 2 | A | B |
| Example 7 | A | 5 | 6 | 10 | A | B | A | 6 | 10 | 14 | B | B |
| Example 8 | A | 0 | 0 | 0 | A | B | A | 0 | 1 | 2 | A | B |
| Example 9 | A | 1 | 4 | 9 | A | B | A | 2 | 5 | 9 | B | B |
| Example 10 | A | 1 | 5 | 8 | A | A | A | 2 | 5 | 9 | A | A |
| Example 11 | A | 2 | 5 | 12 | B | B | B | 6 | 11 | 13 | B | C |
| Example 12 | A | 3 | 5 | 13 | A | B | A | 3 | 5 | 14 | A | B |
| Example 13 | A | 1 | 2 | 4 | B | B | A | 2 | 5 | 11 | B | C |
| Example 14 | A | 2 | 2 | 5 | B | B | A | 2 | 3 | 4 | B | C |
| Example 15 | A | 2 | 3 | 7 | A | A | A | 3 | 5 | 8 | A | A |
| Example 16 | A | 2 | 3 | 8 | A | A | A | 3 | 6 | 8 | A | A |
| Example 17 | A | 1 | 4 | 8 | A | A | A | 2 | 5 | 8 | A | A |
| Example 18 | A | 1 | 3 | 7 | A | A | A | 3 | 6 | 7 | A | A |
| Example 19 | A | 0 | 0 | 0 | A | A | A | 0 | 0 | 0 | A | A |
| Example 20 | A | 0 | 0 | 0 | A | A | A | 0 | 0 | 0 | A | A |
| Example 21 | A | 0 | 0 | 1 | A | A | A | 0 | 0 | 1 | B | A |
| Example 22 | A | 0 | 0 | 0 | B | A | A | 0 | 0 | 2 | B | A |
| Comparative Example 1 | B | 0 | 0 | 8 | B | B | B | 10 | 31 | 45 | C | C |
| Comparative Example 2 | A | 0 | 0 | 10 | B | B | B | 15 | 22 | 63 | D | C |
| Comparative Example 3 | B | 100 | 100 | 100 | B | A | C | 100 | 100 | 100 | B | B |

From the results shown in Table 3 it can be seen that the artificial nail of the present invention is cured by exposure but can be removed easily using an acid aqueous solution, and high adhesion can also be maintained. It can also be seen that the performance of the artificial nail composition of the present invention is degraded less even after forced aging.

### (Example 23)

An artificial nail having a film thickness of about 100 µm (±10 µm) formed as above was coated as with the commercially available Calgel #CG-03 fresh pink (Moga·Brook Co., Ltd.) as a color layer using a flat brush, and irradiation was carried out using a UV lamp (36 W) for 2 minutes. After an uncured portion was wiped with ethanol, coating as a top layer with a commercially available top gel (Moga·Brook Co., Ltd.) using a flat brush was carried out and irradiation was carried out in the same manner with a UV lamp (36 W) for 2 minutes. When the artificial nail thus formed was visually examined, it had completely solidified. The total film thickness of the color layer and the top layer was about 1,000 µm.

In this case also, the same trend was obtained when the same evaluations as in Example 1 were carried out, and it has been found that the artificial nail of the present invention is cured by exposure, and even when it further has an upper layer, peeling with pushing force can be carried out easily using an acid aqueous solution, and high adhesion can also be maintained. It has been found that degradation of performance even after aging is less.

## Claims

1. An artificial nail composition comprising (Component A) an ethylenically unsaturated group- and primary amino group- and/or secondary amino group-containing compound, and (Component B) a photopolymerization initiator.

2. The artificial nail composition according to Claim 1, wherein Component A is a compound represented by Formula (I) and/or a compound represented by Formula (II) 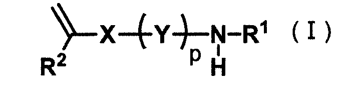 wherein in the Formula R¹ denotes a hydrogen atom, an alkyl group, a cycloalkyl group, or an aryl group, R² denotes a hydrogen atom or a methyl group, p denotes an integer of 0 to 50, X denotes a linking group selected from the group consisting of an alkylene group, -COO-, -CONH-, and -C₆H₄-, Y denotes a divalent linking group, a plurality of Ys may be identical to or different from each other, and Y and R¹ may be bonded to form a ring, 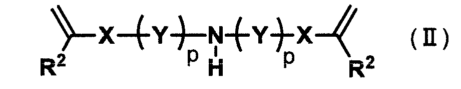 wherein in the Formula R² denotes a hydrogen atom or a methyl group, the two ps independently denote an integer of 0 to 50, X denotes a linking group selected from the group consisting of an alkylene group, -COO-, -CONH-, and -C₆H₄-, Y denotes a divalent linking group, and a plurality of Ys may be identical to or different from each other.

3. The artificial nail composition according to Claim 1 or 2, wherein it is a radiation-curable artificial nail composition.

4. The artificial nail composition according to Claim 2, wherein in Formula (I) and/or Formula (II) X denotes -COO- or -CONH-, and Y denotes an alkylene group, an alkyleneoxy group, an alkylene amide group, or a group formed by combining same.

5. The artificial nail composition according to any one of Claims 1 to 4, wherein Component B is a photopolymerization initiator selected from the group consisting of an acetophenone compound and a phosphine oxide compound.

6. The artificial nail composition according to any one of Claims 1 to 5, wherein it further comprises a film-forming agent.

7. A method for forming an artificial nail, comprising a step of forming a cured film by irradiating the artificial nail composition according to any one of Claims 1 to 6 with actinic radiation.

8. A method for forming an artificial nail, comprising a step of preparing the artificial nail composition according to any one of Claims 1 to 6 and a step of forming a cured film by irradiating the artificial nail composition with actinic radiation.

9. An artificial nail formed by curing the artificial nail composition according to any one of Claims 1 to 6 by irradiation with actinic radiation above a human or animal nail or a synthetic support.

10. A method for removing an artificial nail, comprising a step of removing a cured film of the artificial nail composition according to any one of Claims 1 to 6 by contacting it with an acidic aqueous solution having a pH of no greater than 5.0.

11. A method for removing an artificial nail, comprising a step of preparing a cured film of the artificial nail composition according to any one of Claims 1 to 6 and a step of removing the cured film by contacting it with an acidic aqueous solution having a pH of no greater than 5.0.

12. A nail art kit comprising the artificial nail composition according to any one of Claims 1 to 6 and an acidic aqueous solution having a pH of no greater than 5.0.
